# EUROPÄISCHE PATENTSCHRIFT

(11) **EP 1 042 271 B1**
(45) Veröffentlichungstag und Bekanntmachung des Hinweises auf die Patenterteilung: **05.02.2003**
(21) Anmeldenummer: 98955504.0
(22) Anmeldetag: 21.10.1998
(51) Int. Cl.: C07C 67/48, C07C 69/54, C11D 7/06

(54) **VERFAHREN ZUR REINIGUNG VON ANLAGENTEILEN**
METHOD FOR CLEANING SYSTEM COMPONENTS
PROCEDE DE NETTOYAGE DE PARTIES D'INSTALLATION

(30) Priorität: 22.10.1997 DE 19746688
(43) Veröffentlichungstag der Anmeldung: 11.10.2000
(73) Patentinhaber: BASF AKTIENGESELLSCHAFT, 67056 Ludwigshafen (DE)
(72) Erfinder: AICHINGER, Heinrich, D-68199 Mannheim (DE); HERBST, Holger, D-67227 Frankenthal (DE); NESTLER, Gerhard, A-1070 Wien (AT); SCHRÖDER, Jürgen, D-67071 Ludwigshafen (DE)
(74) Vertreter: Isenbruck, Günter, Dr.
(86) Internationale Anmeldenummer: EP9806677
(87) Internationale Veröffentlichungsnummer: WO99020595

(56) Entgegenhaltungen:
- DE-A- 3 325 166
- PATENT ABSTRACTS OF JAPAN vol. 011, no. 018 (C-398), 17. Januar 1987 & JP 61 192748 A (ASAHI CHEM IND CO LTD), 27. August 1986

## Beschreibung

Die Erfindung betrifft ein Verfahren zur Reinigung von Anlagenteilen, die zur Herstellung oder Verarbeitung von (Meth)acrylsäureestern eingesetzt werden.

(Meth)acrylsäureester werden großtechnisch in der Regel durch Veresterung von (Meth)acrylsäure mit Alkanolen in Gegenwart von starken Säuren und gegebenenfalls eines Schleppmittels zur Entfernung des Veresterungswassers hergestellt. Die Synthese erfolgt vorzugsweise in Rührreaktoren mit aufgesetzten Destillationskolonnen, die Aufarbeitung mit Hilfe von mehreren Destillationskolonnen und Extraktionsapparaturen (vergleiche Kirk Othmer, Encyclopedia of Chemical Technology, 4. Ed., Vol. 1, Seiten 301-302). Die Wärmezufuhr und die Kühlung erfolgen üblicherweise mit Wärmeaustauschern, wie Platten-, Rohrbündel-, Spiralwärmeaustauschern oder Umlaufverdampfern. Die Destillationskolonnen sind mit Einbauten, wie Sieb-, Glocken-, Dual-Flow-Böden oder gerichteten Packungen versehen oder enthalten Füllkörper.

Ein Problem bei der Herstellung von (Meth)acrylsäureestern stellt die große Neigung der (Meth)acrylsäureverbindungen zur radikalischen Polymerisation dar, besonders unter der Einwirkung von Wärme oder Licht. Vor allem bei der Veresterung sind, um wirtschaftliche Veresterungsgeschwindigkeiten zu erreichen, in der Regel Reaktionstemperaturen von 80 bis 140°C notwendig. Bei der Reinigung erfordern die notwendige destillative Abtrennung der Leicht- und Schwersieder und die Isolierung des Zielesters Temperaturen von 60 bis 150°C. Somit sind die Ester Temperaturbelastungen ausgesetzt, die leicht eine unerwünschte Polymerisation auslösen können. Die Verschmutzung der Herstellapparatur, der Destillationskolonnen und die Verstopfung der Leitungen, Pumpen, Verdampfer ("Fouling") und Kondensatoren durch Polymerisate sind meistens die Folge.

Insbesondere beim Einsatz der aus ökonomischen Gründen bevorzugten rohen Acrylsäure, die noch verschiedene Carbonylverbindungen, wie Formaldehyd, Acetaldehyd, Furfurale, Benzaldehyd, Protoanemonin und Dicarbonsäuren, wie Maleinsäure oder Itaconsäure enthält, kommt es vermehrt zur Verschmutzung und Verstopfung von Anlagenteilen.

Um die unerwünschte radikalische Polymerisation zu verhindern, werden dem Reaktionsgemisch Inhibitoren zugesetzt.

Eine vollkommene Eliminierung der Polymerisatbildung ist damit aber nicht erreichbar. Nach mehreren Wochen Laufzeit müssen deshalb in der Regel Anlageteile von Polymerisat befreit werden.

Erschwerend kommt hinzu, daß höhermolekulare Verbindungen, wie sie durch mehrfache Michaeladdition der (Meth)acrylsäure an das (Meth)acrylat entstehen, die Viskosität des Reaktionsgemisches erhöhen und die Ablagerung von Polymerisat in den Anlagenteilen erleichtern.

Die Reinigung der Anlagen, zum Beispiel durch Behandlung mit Lösemitteln oder durch manuelle Entfernung des Polymerisats, ist ein aufwendiger, kostspieliger und umweltbelastender Vorgang. Die Reinigung der Anlagenteile erfolgt üblicherweise durch Behandlung mit geeigneten organischen Lösemitteln, zum Beispiel Dimethyl-, Dibutylformamid, -acetamid, Sulfolan oder N-Methylpyrrolidon. Die dabei anfallenden Lösungen müssen aufwendig entsorgt werden. Zusätzlich kann auch eine manuelle Entfernung des Polymerisats, zum Beispiel von Destillationsböden oder aus Verdampferrohren, notwendig sein.

Neben der Umweltbelastung durch die Reinigung verursachen die Polymerisatbildung und die Reinigung der Anlagenteile immer auch einen Verlust an Wertprodukten, da Restmengen an Ausgangsstoffen und Zielester in der Anlage nach der Entleerung verbleiben. Außerdem wird die Verfügbarkeit der Produktionsanlage durch die notwendige Außerbetriebnahme vermindert.

Aus ökonomischen und ökologischen Gründen ist daher eine andere Reinigung der Anlage und eine weitgehende Vermeidung von Wertproduktverlusten anzustreben.

Aufgabe der vorliegenden Erfindung ist die Bereitstellung eines Verfahrens zur Reinigung von Anlagenteilen, die zur Herstellung oder Verarbeitung von (Meth)acrylsäureestern eingesetzt werden, das die vorstehenden Nachteile vermeidet.

Die Aufgabe wird im erfindungsgemäßen Verfahren gelöst durch
(a) Entleeren der Anlagenteile,
(b) Spülen der Anlagenteile mit wäßriger 5 bis 50 gew.-%iger Alkalihydroxidlösung,
(c) Entfernen der Alkalihydroxidlösung aus den Anlagenteilen,
(d) gegebenenfalls Nachspülen der Anlagenteilen mit Wasser und
(e) gegebenenfalls Trocknen der Anlagenteile.

Vor dem Spülen mit Alkalihydroxidlösung werden die Anlagenteile möglichst vollständig entleert, so daß nur möglichst geringe Mengen an Reaktionsgemisch oder an jeweils im Anlagenteil vorliegender Substanz zurückbleiben.

Die Konzentration der eingesetzten Alkalihydroxidlösung beträgt vorzugsweise 10 bis 30 Gew.-%, bezogen auf das Gewicht der fertigen Lösung.

Vorzugsweise beträgt die Temperatur der Alkalihydroxidlösung beim Spülen 20 bis 140°C, besonders bevorzugt 60 bis 100°C. Es sollte sichergestellt werden, daß die Alkalihydroxidlösung diese Temperatur beim Kontakt mit den abgelagerten Polymerisaten in den Anlagenteilen aufweist. Wird die gesamte Anlage auf die entsprechende Temperatur geheizt, so entspricht die Temperatur der eingesetzten Alkalihydroxidlösung dieser gewünschten Temperatur. Werden Anlagenteile nicht auf diese Temperatur geheizt, so sollte die Temperatur der eingesetzten Alkalihydroxidlösung so eingestellt werden, daß an den Stellen mit Polymerisatbelag die angegebene Temperatur herrscht. Gegebenenfalls wird die Temperatur der Alkalihydroxidlösung beim Einführen in die Anlage entsprechend höher gewählt.

Als Alkalihydroxid wird vorzugsweise Natriumhydroxid verwendet. Die Alkalihydroxidlösung kann durch die entsprechenden Anlagenteile im Kreis gefahren werden, um eine hinreichend lange Behandlungsdauer zu erzielen. Die Behandlungsdauer beträgt dabei je nach Grad der Ablagerungen in den Anlagenteilen üblicherweise 1 bis 24 Stunden. Die Behandlungsdauer, die Konzentration der Alkalihydroxidlösung und die Temperatur richten sich nach der Art des im Anlagenteil verwendeten (Meth)acrylsäureesters und der Art und Menge des Polymerisats. Üblicherweise werden (Meth)acrylsäureester aus Alkoholen, wie Alkanolen mit 1 bis 12 C-Atomen, vorzugsweise 1 bis 8 C-Atomen durch direkte Veresterung oder Umesterung hergestellt. Beispiele für Alkanole sind Methanol, Ethanol, n-Butanol, 2-Ethylhexanol, Dimethylaminoethanol.

Die (Meth)acrylsäureester können auch durch Umsetzung von Olefinen, vorzugsweise Isobuten, mit (Meth)acrylsäure erhalten werden.

Die zu reinigenden Anlagenteile sind solche Anlagenteile, die mit den Ausgangsstoffen oder Produkten bei der Herstellung oder Verarbeitung von (Meth)acrylsäureestern, insbesondere bei der Veresterung von (Meth)acrylsäure mit Alkanolen in Berührung kommen. Bei den Anlagenteilen kann es sich beispielsweise um, gegebenenfalls zu einer Einheit verbundene, Reaktoren, Destillationskolonnen, Extraktionsapparaturen, Wärmeaustauscher, Verdampfer, Kondensatoren, Leitungen oder Pumpen handeln. Vorzugsweise handelt es sich bei den verbundenen Anlagenteilen um eine Destillationseinheit, die eine Spülleitung zwischen Verdampfer und Kolonnenkopf aufweist. In diesen Anlagenteilen lagern sich insbesondere bei der Herstellung und Verarbeitung von (Meth)acrylsäureestern gebildete Polymerisate ab. Die Behandlung mit der Alkalihydroxidlösung bewirkt zumindest teilweise eine Verseifung der Estergruppen der (Meth)acrylsäureester-Polymerisate, so daß es zur Freisetzung der Alkanolkomponente kommt. Unter anderem wird hierdurch das Polymerisat von den Anlagenteilen abgelöst und geht ganz oder teilweise in Lösung und kann so mit der Alkalihydroxidlösung aus den Anlagenteilen ausgetragen werden.

Damit Destillationseinrichtungen mit der Alkalihydroxidlösung gut zu reinigen sind, sind sie vorzugsweise mit eigenen Spülleitungen ausgerüstet. Diese ermöglichen den Transport der Alkalihydroxidlösung, die beispielsweise im Verdampfer der Kolonne erhitzt wird, zum Kopf der Kolonne. Die Spülleitungen erlauben die Behandlung des Kolonnenkopfes und aller Kolonnenböden mit der Alkalihydroxidlösung.

Die bei der Reinigung gebildeten Alkohole, insbesondere Alkanole, werden vorzugsweise nach dem Entfernen mit der Alkalihydroxidlösung aus den Anlagenteilen in Schritt (c) durch Phasentrennung, Destillation oder Strippen von der Alkalihydroxidlösung, in der sie enthalten sind, abgetrennt. Das gebildete Alkanol bildet, falls seine Wasserlöslichkeit gering ist, eine zweite Phase aus, die leicht von der Alkalihydroxidlösung abgetrennt werden kann. Handelt es sich um ein wasserlösliches Alkanol, wird dieses vorzugsweise destillativ oder durch Strippen mit einem Strippgas, wie Luft oder Wasserdampf abgetrennt. Die destillative Abtrennung und das Strippen können beispielsweise in einem beheizbaren Rührreaktor mit einer aufgesetzten Kolonne erfolgen. Der Energieeintrag kann auf bekannte Weise beispielsweise durch Doppelwandheizungen, Rohrschlangen oder Umlauferhitzer erfolgen. Das Strippen des Alkanols kann in einer Strippkolonne auf bekannte Weise erfolgen. Beispielsweise kann die, vorzugsweise heiße, Alkalihydroxidlösung nach dem Spülen am Kopf der Kolonne eingespeist werden und im Gegenstrom mit Luft oder Dampf gestrippt werden. Die Kondensation des Destillats beziehungsweise des Alkanols aus dem Strippgas kann mit bekannten Kühlvorrichtungen, wie Rohrbündel- oder Plattenwärmeaustauschern erfolgen.

Vorzugsweise erfolgt das Strippen in der Strippeinheit, die in der Regel in jeder Anlage zur Herstellung von (Meth)acrylsäureestern vorhanden ist und in der üblicherweise anfallende alkanolhaltige Abwässer gestrippt werden. Der Alkohol, insbesondere das Alkanol, kann nach dem Abtrennen wieder direkt in die Veresterungsreaktion zurückgeführt werden.

Da in der Regel nur ein Teil der eingesetzten Alkalihydroxidlösung bei der Reinigung verbraucht wird, kann die Alkalihydroxidlösung nach der Reinigung aufgefangen und mehrmals zur Reinigung verwendet werden. Dabei sollte die Konzentration an Alkalihydroxid vorzugsweise jedoch nicht unter 5 Gew.-% sinken, um die Reinigungsleistung nicht zu verschlechtern.

Das erfindungsgemäße Verfahren ist einfach und schnell durchführbar, wodurch die Anlagenverfügbarkeit erhöht wird. Zudem ist die Rückgewinnung von Wertprodukten, insbesondere Alkanolen möglich.

Nachstehend wird die Erfindung anhand eines Beispiels näher erläutert.

### Beispiel

### Reinigung einer Destillationskolonne:

Als Destillationskolonne wurde eine Dualflow-Kolonne zur Destillation von Reaktionsgemischen verwendet, die bei der Veresterung von (Meth)acrylsäure mit Alkanolen anfällt. Nach Anzeige der Verschmutzung der Kolonnenböden der Destillationskolonne, erkennbar durch den Anstieg der Druckdifferenz zwischen Kolonnensumpf und Kolonnenkopf, wurde der Destillationsvorgang unterbrochen und die Destillationseinheit entleert. Anschließend wurde der entsprechende Verdampfer mit 20 gew.-%iger wäßriger NaOH-Lösung gefüllt. Die NaOH-Lösung wurde auf eine Temperatur von 80°C erhitzt und über eine Spülleitung auf den obersten Kolonnenboden aufgebracht. Nach einer Behandlungsdauer von 5 Stunden mit dieser heißen Lösung bei einer Umpumprate von etwa 10 m³/h wurde sie in einen Vorratsbehälter gepumpt und die Destillationseinheit mit Wasser gespült und, falls notwendig, mit Luft getrocknet.

Nach erneuter Inbetriebnahme der Destillationskolonne war die Druckdifferenz wieder normal.

### Rückgewinnung des Alkanols:

Eine bei der Reinigung einer Anlage zur Herstellung von Butylacrylat anfallende Spüllösung, die etwa 7 Gew.-% NaOH und etwa 5 Gew.-% Butanol enthielt, wurde mit einer Temperatur von 60°C auf den Kopf einer Strippkolonne, die 30 Dual-Flow-Böden aufwies, gebracht und im Gegenstrom mit Dampf (0,2 t/m³) gestrippt. Das anfallende Kondensat zerfiel in zwei Phasen, wobei die wäßrige Phase wieder auf den Kopf der Kolonne aufgebracht wurde. Die organische Phase, das Butanol, wurde direkt der Veresterung zugeführt. Es wurden etwa 90 % des Butanols zurückgewonnen.

## Patentansprüche

1. Verfahren zur Reinigung von Anlagenteilen, die zur Herstellung oder Verarbeitung von (Meth)acrylsäureestern eingesetzt werden, durch
(a) Entleeren der Anlagenteile,
(b) Spülen der Anlagenteile mit wäßriger 5 bis 50,
gew.-% igerAlkalihydroxidlösung,
(c) Entfernen der Alkalihydroxidlösung aus den Anlagenteilen,
(d) gegebenenfalls Nachspülen der Anlagenteile mit Wasser und
(e) gegebenenfalls Trocknen der Anlagenteile.

2. Verfahren nach Anspruch 1, **dadurch gekennzeichnet, daß** die Temperatur der Alkalihydroxidlösung beim Spülen 20 bis 140°C beträgt.

3. Verfahren nach Anspruch 1 oder 2, **dadurch gekennzeichnet, daß** es sich bei den Anlagenteilen um, gegebenenfalls zu einer Einheit verbundene, Reaktoren, Destillationskolonnen, Extraktionsapparaturen, Wärmetauscher, Verdampfer, Kondensatoren, Leitungen oder Pumpen handelt.

4. Verfahren nach Anspruch 3, **dadurch gekennzeichnet daß** es sich bei der Einheit der verbundenen Anlagenteile um eine Destillationseinheit handelt, die eine Spülleitung zwischen Verdampfer und Kolonnenkopf aufweist.

5. Verfahren nach einem der Ansprüche 1 bis 4, **dadurch gekennzeichnet, daß** die Anlagenteile bei der Herstellung von (Meth)acrylsäureestem durch Umsetzung von (Meth)acrylsäure mit Alkoholen oder Olefinen oder durch Umsetzung von (Meth)acrylsäureestern mit Alkoholen eingesetzt werden.

6. Verfahren nach einem der Ansprüche 1 bis 5, **dadurch gekennzeichnet, daß** die Anlagenteile von bei der Herstellung oder Verarbeitung von (Meth)acrylsäureestern gebildeten, abgelagerten Polymerisaten gereinigt werden.

7. Verfahren nach Anspruch 6, **dadurch gekennzeichnet, daß** nach Schritt (c) in der Alkalihydroxidlösung enthaltene Alkohole durch Phasentrennung, Destillation oder Strippen von ihr abgetrennt werden und gegebenenfalls in die Veresterung zur Herstellung von (Meth)acrylsäureestern zurückgeführt werden.

8. Verfahren nach einem der Ansprüche 1 bis 7, **dadurch gekennzeichnet, daß** die Alkalihydroxidlösung nach der Reinigung aufgefangen und mehrmals zur Reinigung verwendet wird.

9. Verwendung von Alkalihydroxiden zur Reinigung von Anlagenteilen, die zur Herstellung oder Verarbeitung von (Meth)acrylsäureestern dienen.

## Claims

1. A method for cleaning plant parts which are used for the production or processing of (meth)acrylic esters, by
(a) emptying the plant parts,
(b) flushing the plant parts with aqueous 5 to 50% strength by weight alkali metal hydroxide solution,
(c) removing the alkali metal hydroxide solution from the plant parts,
(d) if required, washing the plant parts with water and
(e) if required, drying the plant parts.

2. A method as claimed in claim 1, wherein the temperature of the alkali metal hydroxide solution during flushing is from 20 to 140°C.

3. A method as claimed in claim 1 or 2, wherein the plant parts are reactors, distillation columns, extraction apparatuses, heat exchangers, evaporators, condensers, pipes or pumps, which may be connected to form a unit.

4. A method as claimed in claim 3, wherein the unit comprising the connected plant parts is a distillation unit which has a flushing line between the evaporator and the top of the columns.

5. A method as claimed in any of claims 1 to 4, wherein the plant parts are used in the production of (meth)acrylic esters by reaction of (meth)acrylic acid with alcohols or olefins or by reacting (meth)acrylic esters with alcohols.

6. A. method as claimed in any of claims 1 to 5, wherein the plant parts are cleaned to remove deposited polymers formed in the production or processing of (meth)acrylic esters.

7. A method as claimed in claim 6, wherein alcohols contained in the alkali metal hydroxide solution after step (c) are separated from said solution by phase separation, distillation or stripping and, if required, recycled to the esterification for the production of (meth)acrylic esters.

8. A method as claimed in any of claims 1 to 7, wherein the alkali metal hydroxide solution is collected after the cleaning and used several times for cleaning.

9. The use of alkali metal hydroxides for cleaning plant parts which serve for the production or processing of (meth)acrylic esters.

## Revendications

1. Procédé de nettoyage de parties d'installation, mises en oeuvre pour la préparation ou le retraitement d'esters d'acide (méth)acrylique, par
(a) vidage des parties d'installation,
(b) rinçage des parties d'installation au moyen d'une solution aqueuse à 5 à 50% en poids d'un hydroxyde de métal alcalin,
(c) élimination de la solution d'hydroxyde de métal alcalin des parties d'installation,
(d) éventuellement post-rinçage des parties d'installation à l'eau, et
(e) éventuellement séchage des parties d'installation.

2. Procédé selon la revendication 1, **caractérisé en ce que** la température de la solution d'hydroxyde alcalin lors du rinçage est de 20 à 140°C.

3. Procédé selon la revendication 1 ou 2, **caractérisé en ce que** les parties d'installation sont des réacteurs, des colonnes de distillation, des appareillages d'extraction, des échangeurs de chaleur, des évaporateurs, des condenseurs, des canalisations ou des pompes, éventuellement réunis en une seule unité.

4. Procédé selon la revendication 3, **caractérisé en ce que** l'unité formée par les parties d'installation est une unité de distillation, qui présente une conduite de rinçage entre l'évaporateur et la tête de la colonne.

5. Procédé selon l'une des revendications 1 à 4, **caractérisé en ce que** les parties d'installation sont mises en oeuvre dans la préparation d'esters d'acide (méth)acrylique par réaction d'acide (méth)acrylique avec des alcools ou des oléfines ou par réaction d'esters d'acide (méth)acrylique avec des alcools.

6. Procédé selon l'un des revendications 1 à 5, **caractérisé en ce que** les parties d'installation sont débarrassées des polymères formés et déposés lors de la préparation ou du retraitement d'esters d'acide (méth)acrylique.

7. Procédé selon la revendication 6, **caractérisé en ce que**, après l'étape (c), les alcools contenus dans la solution d'hydroxyde alcalin en sont séparés par séparation de phases, distillation ou stripage, et sont éventuellement recyclés dans l'estérification pour la préparation d'esters d'acide (méth)acrylique.

8. Procédé selon l'une des revendications 1 à 7, **caractérisé en ce que** la solution d'hydroxyde alcalin est recueillie après le nettoyage et réutilisée plusieurs fois pour le nettoyage.

9. Utilisation d'hydroxydes alcalins pour le nettoyage de parties d'installation, servant à la préparation ou au retraitement d'esters d'acide (méth)acrylique.
